# EUROPEAN PATENT SPECIFICATION

(11) **EP 1 617 812 B1**
(45) Date of publication and mention of the grant of the patent: **06.10.2010**
(21) Application number: 04757952.9
(22) Date of filing: 22.03.2004
(51) Int. Cl.: A61K 8/37, A61K 8/42, A61Q 13/00

(54) **PHOTORESPONSIVE FRAGRANCES**
AUF LICHT REAGIERENDE DUFTSTOFFE
PARFUMS PHOTOSENSIBLES

(30) Priority: 21.03.2003 US 456877 P; 25.03.2003 US 457232 P
(43) Date of publication of application: 25.01.2006
(73) Proprietor: Pho Derma, Inc., Portland, OR 97201 (US)
(72) Inventor: STOWELL, Michael, H., B., Boulder, CO 80305 (US)
(74) Representative: Viering, Jentschura & Partner
(86) International application number: PCT/US2004/008610
(87) International publication number: WO 2004/084853

(56) References cited:
- EP-A- 0 826 764
- EP-A- 0 952 142
- WO-A-99/07336
- WO-A-02/083620
- FR-A- 2 833 259
- GB-A- 1 488 061
- US-A- 5 767 288
- DATABASE CHEMABS [Online] CHEMICAL ABSTRACTS SERVICE, COLUMBUS, OHIO, US; 1979, SASAKI, KUNITAKA ET AL: "Solid perfume compositions" XP002296462 retrieved from STN Database accession no. 1979:444408 & JP 53 146744 A2 (INSHA Y. K., JAPAN) 20 December 1978 (1978-12-20)
- M.C.PIRRUNG ET.AL.: "Photoremovable Protecting Groups for Phosphorylation of Chiral Alcohols. Asymmetric Synthesis of Phosphotriesters of (-)-3',5'-dimethoxybenzoin." JOURNAL OF ORGANIC CHEMISTRY, vol. 59, 1994, pages 3890-3897, XP002296460 cited in the application
- J.C.SHEEHAN ET. AL.: "The Photolysis of Methoxy-Substituted Benzoin Esters. A Photosensititve Protecting Group for Carboxylic Acids." JOURNAL OF THE AMERICAN CHEMICAL SOCIETY, vol. 93, no. 26, 1971, pages 7222-7228, XP002296461 cited in the application

## Description

### BACKGROUND OF THE INVENTION

### 1. Field of the Invention

This invention relates to photoresponsive fragrance compositions, and more particularly, to compositions that can be applied to textiles, skin, and hair, which produce a pleasant smell when exposed to sunlight. The invention provides such photosensitive fragrance compositions comprising a carrier for topical administration or direct administration or via routine washing, and an organic photoresponsive fragrance agent capable of undergoing efficient photorearrangement to convert the agent molecules and release a desired fragrance.

### 2. Background of the Related Art

The application of a fragrance for scenting textiles is routinely performed during washing of such textiles. Detergents used for these purposes contain a variety of fragrance molecules to give a pleasant smell.

Effective fragrances must be volatile in order that they may be scented. This requirement poses a storage issue in that textiles will not retain fragrances for long periods of time, for example during traveling or storage in a closet or otherwise.

In addition lotions, body washes, and shampoos commonly contain fragrances, where the lotions, body washes and shampoos lose scent over time.

Therefore, there is a need in this art for more efficient methods for imparting fragrances to textiles, clothing and other materials in a fashion that increases the duration of time in which the materials retain a desired scent. There is a further need in the art for fragrance application and impregnation methods and compositions that enable the scented textile to be stored for extended periods of time and for the fragrance to be released at a desired time. In such a manner textiles can be stored for long periods of time and still maintain a just washed freshness in scent. There is also a need for more efficient methods for scenting hair and body lotions, soaps and shampoos, to produce hair or skin that maintains a "just washed" scent following routine washing with soaps and shampoos or treatment with lotions.

### SUMMARY OF THE INVENTION

This invention provides compositions that contain nonvolatile fragrances that are released upon exposure to sunlight. In such a manner textiles can be stored for extended periods of time and then upon modest exposure to sunlight will begin releasing a desired fragrance. In such a manner textiles can be stored for long periods of time and still maintain a just washed freshness in scent.

This invention further provides cosmetic products including lotions, soaps, body washes, and shampoos formulated so that fragrances are continually released following routine washing so that skin and hair maintain a fresh smell.

The invention provides photoactive compounds as ingredients in photoresponsive fragrance compositions described herein. In particular, photoresponsive fragrance compositions are provided for application to clothes, textiles, skin and hair, comprising a nonvolatile carrier for administration during routine washing or as a spray application. In one embodiment, the photoresponsive fragrance compositions of the invention comprise derivatives of 3',5'-dimethoxybenzoin having the structure: 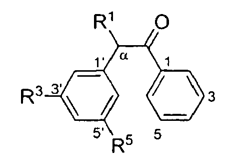 wherein R¹, R³, and R⁵ are as defined below.

Specific preferred embodiments of the present invention will become evident from the following more detailed description of certain preferred embodiments and the claims.

### BRIEF DESCRIPTION OF THE DRAWINGS

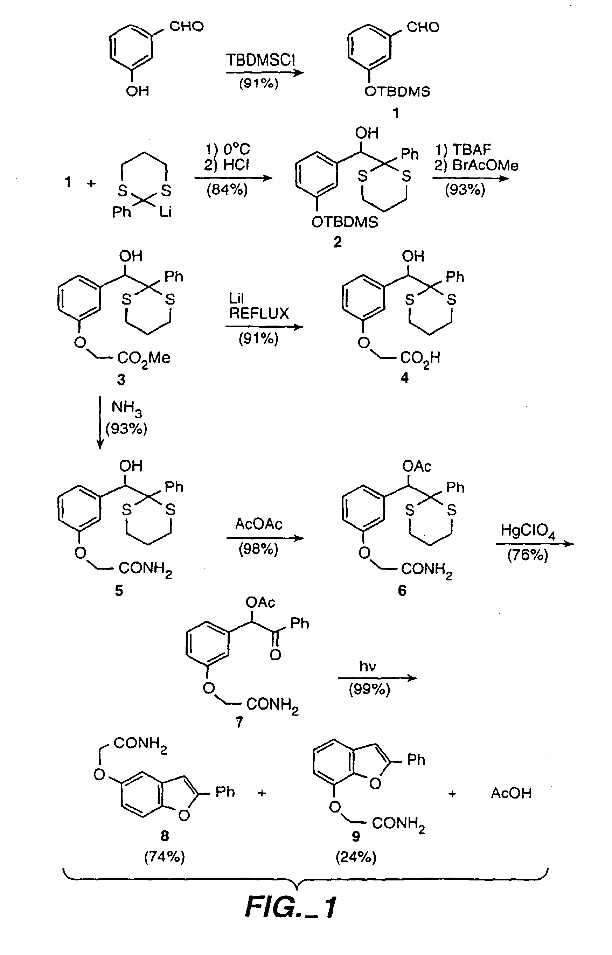

FIG. 1 depicts the synthesis of exemplary compounds of the invention.

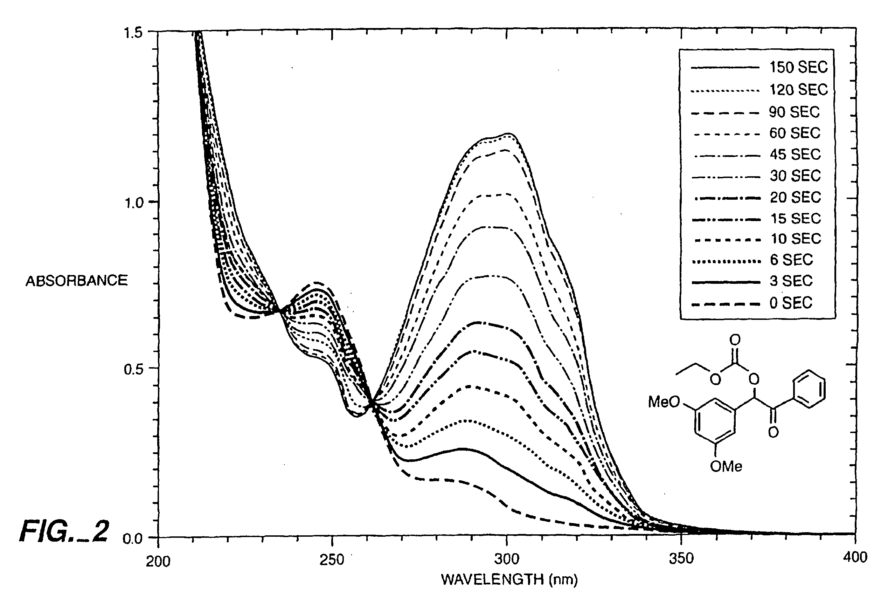

FIG. 2 is an ultraviolet spectrograph showing the steady-state photolysis of 3',5'-dimethoxybenzoin ethyl carbonate from 0 to 150 seconds. A 53.5 microM solution of the benzoin derivative in acetonitrile was irradiated with an Oriel 66011 Hg vapor lamp using UG 11 and WG320 filters. These results demonstrated the efficiency of the photoconversion and release of the desired fragrance.

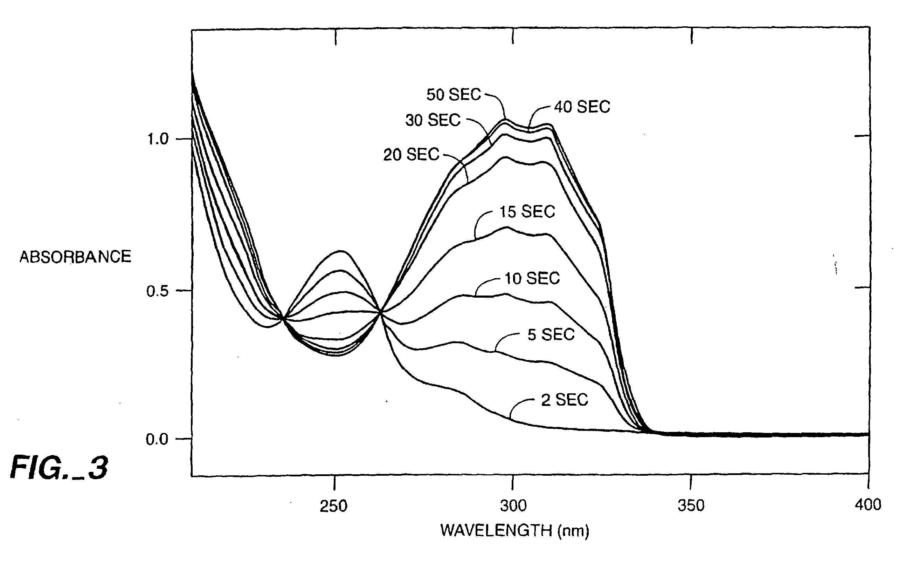

FIG. 3 is an ultraviolet spectrograph showing the steady-state photolysis of (±)-O-acetyl-3'-carbamylmethoxybenzoin. A 54.8 microM solution of (±)-O-acetyl-3'-carbamylmethoxybenzoin in 1:1 methanol/Tris-HCl (0.05 M, pH 7.4) was irradiated with an Oriel 66011 Hg vapor lamp using UG11 and WG320 filters. These results further demonstrated the efficiency of the photoconversion.

### DETAILED DESCRIPTION OF THE PREFERRED EMBODIMENTS

This invention provides photoresponsive fragrance compositions and methods of use thereof for treating textiles, skin and/or hair of mammals (especially humans or companion animals such as dogs, cats, hamsters, gerbils, etc.) and other materials to impart a fragrance thereto. The photoresponsive fragrance compositions of the invention comprise a photoresponsive fragrance agent and a carrier for administration.

Described herein is the novel use of photoresponsive compounds as new fragrance agents. Preferred photoresponsive fragrance agents are capable of undergoing intramolecular photorearrangements to release volatile fragrance. These photolabile compounds can be used in textile washing compositions to provide at least one element whose volatile fragrance increases with the amount of light present. Likewise the photoresponsive fragrance agents can be utilized in body lotions, soaps and shampoos to provide at least one element whose volatile fragrance increases with the amount of light present. The photoresponsive fragrance agents can be used alone or in combination with known fragrance agents.

Many photolabile compounds are available. Compounds having photolytic properties, including benzoin esters of carboxylates and phosphates (Corrie et al., 1992, J. Chem. Soc. Perkin. Trans. 1: 2409; Pirrung et al., 1994, J. Org. Chem. 59: 3890) have been described and are known in the art.

One class of photolabile compounds useful in the compositions and practice of the methods of this invention are substituted benzoins (as initially reported by Sheehan et. al., 19971, J. Am. Chem. Soc. 93: 7222). Of particular interest are the substituted alkoxybenzoins, having particularly advantageous photocleavage properties for the practice of this invention. Included in this class of compounds are the 3',5'-dimethoxybenioin esters (3',5'-DMB) that undergo a photoinitiated cyclization and cleavage. This reaction has a rate constant estimated to be greater than 10¹⁰ sec⁻¹ and a quantum efficiency of 0.64, when RO- on the α-carbon is acetyl. FIG. 2 shows steady-state photolysis results for 3',5'-dimethoxybenzoin ethyl carbonate. FIG. 3 shows steady-state photolysis results for O-acetyl-3'-carbamylmethoxybenzoin.

In a preferred embodiment, the compositions comprise a first nonvolatile fragrance agent that undergoes intramolecular photorearrangement, initiated by ultraviolet radiation, preferably between about 250 and about 400 nm, releasing a volatile fragrance agent. In a preferred embodiment, photorearrangement rates and therefore release of fragrance are adjusted by including additives that compete for light.

Preferred fragrance compositions comprise photoresponsive fragrance agents and a carrier for topical administration, thereby aiding in the application of appropriate amounts of fragrance agent to the skin, hair or textiles. For topical application, fragrance compositions are preferably nontoxic and nonirritating to the skin tissue. For textiles applications preferred embodiments can be applied during routine washing.

Preferred topical compositions may be in the form of creams, gels, lotions, oils, soaps, shampoos or other solutions comprising as the active agents prephotolysis compounds (Formula 3), post-photolysis compounds (Formula 4), or a combination of the two. The compositions are preferably formulated using homogeneous solutions, such as anhydrous solvents, or heterogeneous mixtures, such as emulsions. Topical carriers and general methods of formulation and manufacture of sunscreen such compositions are known in the art. *See, for example,* U.S. Pat. Nos. 4,522,807 and 4,822,600, both of which are expressly incorporated herein by reference.

Examples of suitable topical carriers are, but are not limited to, water, lower monoalcohols as well as their mixtures, or aqueous alcoholic or oil/alcohol solutions, the preferred alcohols including ethanol, isopropyl alcohol, propylene glycol, glycerol, and sorbitol, and the preferred aqueous alcoholic mixtures being mixtures of water and ethyl alcohol.

In addition to a carrier element to aid in distribution of the photoresponsive fragrance agent onto the skin or hair, some embodiments may include additives to improve the cosmetic properties of the fragrance composition. Some cosmetic ingredients that can be advantageously incorporated into the compositions of this invention include but are not limited to thickeners, softeners, superfatting agents, waterproofing agents, emollients, wetting agents, and surface-active agents, as well as preservatives, anti-foam agents, fragrance, or any other compatible ingredient usually employed for cosmetics. Certain of these additives and components are also useful in embodiments provided for imparting scent to textiles.

Film-forming agents and cosmetic resins are also useful in the practice of the present invention, including for example polyvinylpyrrolidone; vinylpyrrolidone/vinyl acetate copolymers, wherein monomers ratios range from about 70:30 to about 30:70; vinyl acetone/unsaturated carboxylic acid copolymers such as a copolymer containing 90% of vinyl acetate and 10% of crotonic acid, terpolymers of methyl methacrylate/stearyl methacrylate/dimethylaminoethyl methacrylate, completely quaternised with dimethyl sulfate, wherein useful monomers are advantageously included in monomer ratios of about 20:23:57, and a terpolymer of vinyl acetate/allyl stearate/allyloxyacetic acid, especially in the ratio of 80:15:5, maleic anhydride/methyl vinyl ether copolymers such as those commercially referred to as "Gantrex AN" as well as the ethyl, isopropyl, and butyl esters of these copolymers, and maleic anhydride/butyl vinyl ether copolymers.

In addition compositions optimized for use and application to textiles during routine washing are desired. These include compositions containing detergents and enzymes optimized for such cleaning purposes.

In addition to the carrier for administration, photoresponsive fragrance compositions may comprise individual photoresponsive fragrance agents, combinations of two or more photoresponsive fragrance agents of the invention, or combinations of one or more photoresponsive fragrance agents with one or more known fragrance agents.

The photoresponsive fragrance agents of this invention may be combined with one or more known fragrance agents. Preferable known fragrance agents include but are not limited to: Angelica root oil, Anisylidene acetone (4-(4-methoxphenyl)-3-buten-2-one), Asarone ((E)-and(Z)-2,4,5-Trimethoxypropen-1-yl benzene), Benzylidene acetone (4-Phenyl-3-buten-2-one), Bergamot oil expressed, Birch wood pyrolysate, Bitter Orange Peel Oil Expressed, Butyl-dihydrocinnamaldehyde (Bourgeonal), Butylphenol, Cade oil, Carvone oxide, Cassia oil, Cinnamic alcohol, Cinnamic aldehyde, Cinnamic aldehyde - Methyl anthranilate Schiff base, Cinnamon bark oil, Ceylon, Cinnamyl Nitrile, Citral (Lemarome), Citrus oils and other furocoumarins containing essential oils, Colophony, Costus root oil, absolute and concrete, Cumin oil ,Cyclamen alcohol (3-(4-Isopropylphenyl)-2-methylpropanol), Diethyl maleate, Dihydrocoumarin (Melilotine), Dihydroxy-3-methyl-benzaldehyde, Dimethyl-8-t-butyl coumarin (Butolia), Dimethylcitraconate (cis-Methylbutenedioic acid, dimethyl ester), Ethyl acrylate, Farnesol, Fig leaf absolute, Grapefruit oil expressed, Heptenal, Hexahydrocoumarin, Hexenal, Hexenal diethyl acetal, Hexenal dimethyl acetal, Hexylidene cylcopentanone, Hydroabietyl alcohol (Abitol), Hydroquinone monoethylether (4-Ethoxy phenol), Hydroquinone monomethylether (4-Methoxy phenol), Hydroxycitronellal (Laurine, Hydronal, Phixia, Laurinal), Isocyclogeraniol (2,4,6-Trimethyl-3-cyclohexene-1-methanol), loeugenol, Isopropyl-2-decalol, Lemon oil cold pressed, Lime oil expressed, Limonene, Menthadienyl formate (Isobergamate), Methoxy dicylopentadiene carboxaldehyde (Scentenal), Methoxy-4-methylphenol (Creosol), Methoxycoumarin, Methyl crotonate, Methyl heptadienone (6-Methyl-3,5-heptadienone), Methyl heptine carbonate (MHC, Folione), Methyl N-methyl anthranilate (Dimethyl anthranilate), Methyl octine carbonate (MOC),Methyl-2(3)-nonenenitrile (Citgrenile), Methyl-7-ethoxycoumarin (Maraniol), Methylcoumarin, Methylcoumarin (Toncarine), Methyleugenol, Methylhydrocinnamic aldehyde, Musk ambrette, Nitrobenzene, Nootkatone, Oak moss extracts, Octen-3-yl acetate (Amyl vinyl carbinyl acetate), Opoponax, Other materials, Pentylidene cyclohexanone, Perilla aldehyde, Peru balsam, Petitgrain Mandarin Oil, Phenylacetaldehyde (Hyacinthin), Pinacea derivatives, Propylidene phthalide, Pseudoionone (2,6-Dimethylundeca-2,6,8-trien-10-one), Pseudomethylionones, Rue oil, Safrole, Isosafrole, Dihydrosafrole, Savin oil, Sclareol, Styrax, Tagetes oil and absolute, Tree moss extracts, Trimethylcyclohexa-1,3-dienyl methanal (Safranal), Trimethylcyclohexenyl/cyclohexadienyl)-2-buten-1-ones (Rose ketones), Verbena absolute, and Verbena oil.

Preferably, fragrance agents of the invention, such as the benzoin derivatives of Formula 3, should be stored in a dark container to prevent premature photo-rearrangement. Most preferably, such compositions containing benzoin derivatives should be stored in a dark, preferably black, container that significantly limits exposure of the composition to light.

As stated above, benzoin compounds are sensitive to light. Therefore it is helpful to have a stable form of the compound for storage purposes. Benzoin compounds are preferably protected from light by storage as their dithiane derivatives (Formula 2). The dithiane protecting group can be removed by contact with mercuric perchlorate, or via other methods known to those skilled in the art. Removal of the dithiane protecting group gives the benzoin compound (Formula 3). The benzoin compound is converted to the benzofuran compound (Formula 4) by exposure to light.

The generic dithiane adducts depicted in Formula 2, below, represent important, nonphotolabile, intermediate compounds.

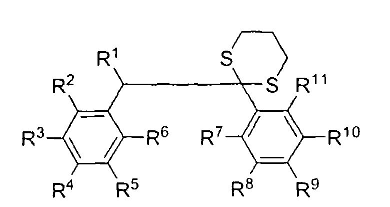

In one embodiment, R₁ is hydroxy, carbonate, substituted ester (--OC(O)R), a phosphorus-containing group, a sulfur-containing group, a fluorescent label, or a fragrance molecule or a molecule that imparts a fragrance. R₂, R₃, R₄, R₅, R₆, R₇, R₈, R₉, R₁₀, and R₁₁ are each hydrogen, alkyl, aryl, alkoxy, substituted alkoxy, or halide. At least one of R₂ or R₆ is hydrogen. At least one of R₂, R₃, R₄, R₅ and R₆ is hydroxy or substituted alkoxy.

The corresponding photolabile benzoin compounds are as generically depicted below in Formula 3:

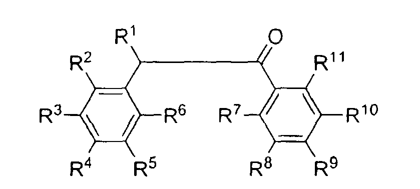

In this embodiment, R₁ is a fragrance, R₂, R₃, R₄, R₅, R₆, R₇, R₈, R₉, R₁₀, and R₁₁ are each hydrogen, alkyl, aryl (such as phenyl), alkoxy, substituted alkoxy, or halide. At least one of R₂ or R₆ is hydrogen. At least one of R₂, R₃, R₄, R₅ and R₆ is hydroxy or substituted alkoxy.

The generic compound depicted in Formula 3 is generally made by removing the dithiane adduct to form the ketone. This is accomplished as is generally known in the art, using mercuric perchlorate or bis(trifluoroacetoxy)-iodobenzene; *see for example* Greene et al. (1991, PROTECTIVE GROUPS IN ORGANIC SYNTHESIS, John Wiley and Sons, New York, pp. 203-205, hereby incorporated by reference). In this manner, the dithiane adduct serves as a protecting group of the photolabile benzoin, during subsequent manipulations or until photolysis is desired. At the appropriate time, the dithiane adduct is removed, and then photolysis is initiated as needed.

In another embodiment, the present invention includes the corresponding photorearranged benzofuran compounds as generally depicted in Formula 4:

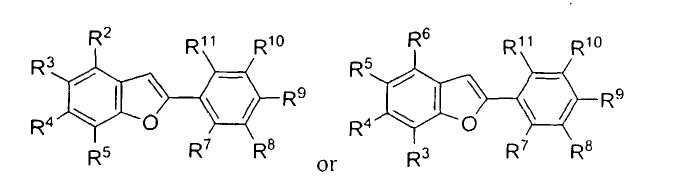

Isomeric compounds 4A and 4B of Formula 4 differ only in that the furan ring formation occurs at the R₆, and R₂ positions, respectively. Where, in the benzoin precursor, R₂ is hydrogen and R₆ is not hydrogen, 4B will be formed. Where, in the benzoin precursor, R₆ is hydrogen and R₂ is not hydrogen, 4A will be formed. Where, in the benzoin precursor, both R₂ and R₆, are hydrogen, a mixture of 4A and 4B will be formed. In this last case, 4A and 4B will be identical compounds unless R₃ and R₅ in the benzoin precursor are different (see FIG. 1, compounds 8 and 9).

In this embodiment R₂, R₃, R₄, R₅, R₆, R₇, R₈, R₉, R₁₀ and R₁₁ are each hydrogen, alkyl, aryl, alkoxy, substituted alkoxy, or halide. At least one of R₂, R₃, R₄, R₅ and R₆ is hydroxy or substituted alkoxy.

The furan products (Formula 4) are formed from the substituted benzoins after exposure to light. Formula I depicts the furan formed by attack at the R₂ (4B) or R₆ (4A) positions, one of which are necessarily hydrogen.

The following functional group definitions further describe the preferred embodiments of the structures described herein.

By "carbonate" herein is meant --OC(O)OR₁₂ group, where R₁₂ is a fragrance or a molecule that imparts a fragrance.

By "substituted ester" herein is meant a --OC(O) R₁₂ group, where R₁₂ is a fragrance or molecule that imparts a fragrance. Thus, the R₁ may be a carbamate of the formula --OC(O)NR'R", where R' and R" may be the same or different and include hydrogen, alkyl, and aryl. When R' is a substituted ester, and R₁₂ is fragrance or molecule that imparts a fragrance.

By "alkyl" or "alkyl group" or grammatical equivalents herein is meant a straight or branched chain alkyl group, with straight chain alkyl groups being preferred. If branched, it may be branched at one or more positions, and unless specified, at any position. Also included within the definition of an alkyl group are cycloalkyl groups such as C5 and C6 rings. In some cases, two R groups may be part of the same ring structure, that is, they may be linked to form a cyclic structure, including heterocyclic structures. For example, R₃ and R₄ and/or R₉ and R₁₀ may be joined to form a methylene dioxy, five-membered ring (as described in Pillai, Synthesis, January 1980, pp. 1-26, incorporated herein by reference); in addition, R₂ and R₃, and/or R₇ and R₈, may also be similarly joined. In some cases, the R groups may form an aryl group; for example, R₉ and R₁₀ may form a benzyl group, such that a naphthyl group is formed (as described in U.S. Pat. No. 4,469,774, incorporated by reference).

Alkyl groups as disclosed as substituents of the formulae of this invention may range in size from about 1 to 100 carbon atoms (C₁-C₁₀₀), with a preferred embodiment utilizing from about 1 to about 20 carbon atoms (C₁-C₂₀), with about C₁ through about C₈ being most preferred. However, in some embodiments, the alkyl group may be larger, particularly if it is a straight chain alkyl. Particularly preferred compounds have methyl groups in the R₂ to R₆ positions.

By "aryl" or "aryl group" herein is meant aromatic rings including phenyl, benzyl, and naphthyl, as well as heterocyclic aromatic rings such as pyridine, furan, thiophene, pyrrole, indole and purine, and other heterocyclic aromatic rings containing carbon, nitrogen, oxygen, sulfur, or phosphorus.

The alkyl and aryl groups as disclosed as substituents of the formulae of this invention may be substituted; for example, a phenyl group may be a substituted phenyl group. Suitable substitution groups include, but are not limited to, alkyl groups; alkoxy groups; OCF₃; CF₃; aryl groups, such as phenyl; halogens such as chlorine, bromine and fluorine; amines; carboxylic acids; amides, amines, and nitro groups.

By the term "amine" herein is meant an -NR₁₃R₁₄ group. In this embodiment, R₁₃ and R₁₄ may be the same or different, and may be hydrogen, alkyl or aryl (such as phenyl or naphthyl.) A preferred -- NR₁₃R₁₄ group is --NH₂. A secondary amine is -NR₁₃R₁₄ where either R₁₃ or R₁₄, but not both, is hydrogen. A tertiary amine is -NR₁₃R₁₄ where neither R₁₃ nor R₁₄ is hydrogen.

By "hydroxy" herein is meant an --OH group.

By "alkoxy" herein is meant an -OR₁₅ group, where R₁₅ is an alkyl group as defined above. Included within the definition of alkoxy is methoxy (--OCH₃).

By "substituted alkoxy" herein is meant a --OXC(R₁₆)(R₁₇)(R₁₈) group, wherein X is either not present (*i.e*. substituted methoxy) or a straight or branched chain alkyl group. In a preferred embodiment, X is a straight chain alkyl group, such that the substituted alkoxy group has the formula --O(CH₂)ₙC(R₁₆)(R₁₇)( R₁₈), wherein n is 0 (substituted methoxy, which is preferred) or greater, preferably from 0 to 100, with 0 to 20 being especially preferred. R₁₆, R₁₇ and R₁₈ are selected from the group consisting of hydrogen, amino, carboxy, phosphorus-containing groups, sulfur-containing groups, protecting groups such as silyl groups and others known in the art, a fragrance or molecule that imparts a fragrance. In a preferred embodiment, R₁₆ and R₁₇ are hydrogen, such that there is a single substitution group.

By "phosphorus-containing group" herein is meant a functional group containing at least one phosphorus atom. In a preferred embodiment, the phosphorus-containing group is chemically or functionally active, such that further groups may be attached to the compound using the phosphate. In a preferred embodiment, the phosphorus-containing group is a phosphate (--OP(O)(OH)₂) group, a pyrophosphate group, or a substituted phosphate group of the Formula-OP(O)(OR₁₉)(OR₂₀). In this embodiment, R₁₉ and R₂₀ include, but are not limited to, hydrogen, alkyl, or aryl (such as phenyl.) In a preferred embodiment, one of R₁₉ and R₂₀ is hydrogen. Also included within the definition of phosphorus-containing groups are phosphines (--RPR₁₉R₂₀), and phosphonates (--R-P(O)(OR₁₉)(OR₂₀)).

By "sulfur-containing group" herein is meant a functional group containing at least one sulfur atom. As for the phosphates, the sulfur-containing group is preferably chemically or functionally active, such that further groups such as a fragrance or molecule that imparts a fragrance may be attached using the sulfur atom. Thus thiols (--RSH), sulfides (--RSR₁₉), sulfoxides (--RS(O)R₁₉), sulfones (--RS(O)₂R₁₉), sulfates (--ROS(O)₂OR₁₉), and sulfonic acids (--RS(O)₂OH) are all included within the definition of sulfur-containing groups.

By "halide" herein is meant a halide atom. Preferred halides include chlorine, fluorine, bromine, and iodine, with chlorine and fluorine being particularly preferred, and chlorine being most preferred.

As disclosed above, compounds of Formulas 2, 3, and 4 can have many possible structures varying by the functionality of the substituents used. Preferred R groups for Formulas 2, 3, and 4 are as follows:
R₁ is a fragrance or molecule that imparts a fragrance. Preferably, R₁ is attached via a carboxyl, phosphate, sulfate, thiol, or amine (--NR₁). More preferably, R₁ is --OC(O)OR₁₂., where R₁₂ is a fragrance or molecule that imparts a fragrance.
R₂ can be hydrogen, alkyl, aryl (such as phenyl), alkoxy, or substituted alkoxy. Preferably, R₂ is hydrogen.
R₃ can be hydrogen, alkyl, aryl (such as phenyl), alkoxy, or substituted alkoxy. Preferably, R₃ is alkoxy, substituted alkoxy, or -- if R₅ is alkoxy or substituted alkoxy - R₃ is hydrogen- More preferably R₃ is --OMe.
R₄ can be hydrogen, alkyl, aryl (such as phenyl), alkoxy, or substituted alkoxy. Preferably, R₄ is hydrogen.
R₅ can be hydrogen, alkyl, aryl (such as phenyl), alkoxy, or substituted alkoxy. Preferably, R₅ is alkoxy or substituted alkoxy. More preferably R₅ is-OMe.
R₆ can be hydrogen, alkyl, aryl (such as phenyl), alkoxy, or substituted alkoxy. Preferably, R₆ is hydrogen.
R₇ can be hydrogen, alkyl, aryl (such as phenyl), alkoxy, or substituted alkoxy. Preferably, R₇ is hydrogen.
R₈ can be hydrogen, alkyl, aryl (such as phenyl), alkoxy, or substituted alkoxy. Preferably, R₈ is hydrogen.
R₉ can be hydrogen, alkyl, aryl (such as phenyl), alkoxy, or substituted alkoxy. Preferably, R₉ is hydrogen.
R₁₀ can be hydrogen, alkyl, aryl (such as phenyl), alkoxy, or substituted alkoxy. Preferably, R₁₀ is hydrogen.
R₁₁ can be hydrogen, alkyl, aryl (such as phenyl), alkoxy, or substituted alkoxy. Preferably, R₁₁ is hydrogen.

In one aspect, at least one of R₂ and R₆ in Formulas 2 and/or 3 are hydrogen. This is required due to the photolysis mechanism depicted in Equation 1, wherein a furan ring is formed by attack at either the R₂ or R₆ position. Accordingly, at least R₂ or R₆ must be hydrogen; *i.e*. either R₂ or R₆ must not contain a substitution group. In a preferred embodiment, both R₂ and R₆ in Formulas 2 and 3 are hydrogen.

The dithiane-benzoin adducts of Formula 2, and the substituted benzoin compounds of Formula 3 are generally synthesized using the scheme depicted in FIG. 1. Generally, the synthesis comprises contacting the reactants depicted below in Formula 5 (for the formation of Formula 2 compounds):

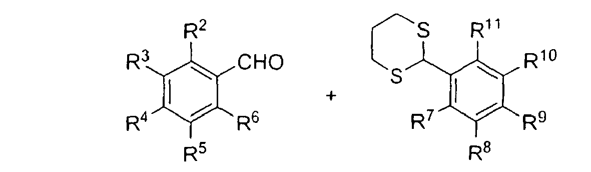

This is done under conditions that allow the formation of the dithiane-benzoin adducts described herein.

Generally, the hydroxy group of hydroxybenzaldehyde is protected, using a known protecting group such as a tert-butyldimethylsiloxy group. A dithiane adduct can be added, using the Corey-Seebach dithiane addition, to form a dithiane-benzoin compound (FIG. 1, Compound 2). The hydroxyl-protecting group is removed, and a chemically-active group is added. For example, a substituted alkoxy can be added. The substituted alkoxy can be altered to include a chemically reactive group such as a carboxy group (FIG. 1, Compound 4), or a carbamate (FIG. 1, Compound 5) at one of the R₂ through R₆ positions. In a similar manner an amino group, a phosphorus-containing group, a sulfur-containing group, a substituted carbonyl, a fragrance molecule or a molecule that imparts a fragrance, a label, or others may be added to the benzyl ring, as is known in the art.

When a fragrance molecule or molecule that imparts a fragrance is to be added to the benzyl or benzoyl ring, or at the R₁ position, it may be done in a variety of ways depending on the fragrance molecule. Generally, when a fragrance is to be attached to the core compound, it is done in two stages. First, the core compound is made containing two chemically active groups; one at the R₁ position, and one at one of the other R groups. For example, the core compound is made with amines, carboxy groups, phosphate groups, or sulfhydryl groups. Next, the fragrance is made, which also contains a functional group that can be used for attachment. In the preparation of some embodiments of the compositions of this invention, other reactive groups of the fragrance agent are protected to prevent them from reacting with the functional group of the core compound. For example, amino groups, may need to be protected to prevent this group from reacting, although in some embodiments the attachment is done *via* a functional group of an amino group. Protecting groups and techniques are well known in the art. Once the core compound and the fragrance molecule or molecule that imparts a fragrance is made, they can be attached by reacting with the functional groups.

The following Examples illustrate certain aspects of the above-described method and advantageous results. The following examples are shown by way of illustration and not by way of limitation.

### EXAMPLES

### General Methods

THF was refluxed over sodium and benzophenone, and was distilled prior to use. 3-hydroxybenzaldehyde (Fluka) was dissolved in diethyl ether, filtered through a plug of neutral alumina, and evaporated. All other starting materials were from Aldrich and used without further purification. The 1.0 M tetrabutylammonium fluoride (TBAF) solution in THF was dried over 3Å molecular sieves. TR spectra were acquired from a thin film of the sample on a polyethylene substrate.

### Example 1

### Synthesis of 3-(tert-butyldimethylsilyloxy)benzaldehyde (FIG. 1, Compound 1):

The hydroxyl of 3-hydroxybenzaldehyde was protected as the tertbutyldimethylsilyl (TBDMS) ether, to circumvent dianion solubility problems. To a solution of 3-hydroxybenzaldehyde (12.21 g, 100 mmol) in 600 mL THF was added t-butyldimethylsilyl chloride (TBDMSCl, 18.84 g, 125 mmol). The solution was cooled to 0° C. and triethylamine (12.65 g, 17.4 mL, 125 mmol) was added dropwise. The reaction mixture was brought to room temperature and stirred 5 hours. The mixture was filtered and the THF removed under reduced pressure. The oil was repeatedly dissolved in 200 mL portions of THF and evaporated until no more triethylamine hydrochloride precipitated. The oil was then dissolved in 150 mL diethyl ether, filtered through a plug of neutral alumina and activated charcoal to remove the salt and the yellow color, and evaporated.

The colorless, mobile oil was dried in vacuo overnight. Yield: 21.43 g (91%). IR: 1703, 1583, 1482, 1278, 1145, 840 cm^{-1 1}H NMR (CDCl₃, TMS) d 9.927 (s, 1 H), 7.447 (d, J=7.50 Hz, 1 H), 7.379-7.335 (m, 2 H), 7.096-7.074 (m, 1 H), 0.994 (s, 9 H), 0.215 (s, 6 H). ¹³C NMR (CDCl₃, TMS) d 191.60, 156.34, 138.03, 130.03, 126.34, 123.46, 119.70, 25.59, 18.12, -4.52. Anal. Calcd for C₁₃H₂₀O₂ Si: C, 66.05; H, 8.53. Found: C, 66.13; H, 8.53.

### Example 2

### Synthesis of (±)-1-hydroxy-1-(3-tert-butyldimethylsilyloxyphenyl)-2-phenyl-2-(1,3-di thian-2-yl)-ethane (FIG. 1, Compound 2):

A solution of 2-phenyl-1,3-dithiane (15.71 g, 80 mmol) in 125 mL THF was prepared. The solution was treated at 0° C. under a nitrogen atmosphere with 40 mL of n-butyllithium (2.0 M in cyclohexane, 80 mmol). After 30 min, 3-(tert-butyl-dimethylsilyloxy)benzaldehyde (18.91 g, 80 mmol) was added. The solution was stirred for 1 hr at 0° C., then poured into 100 mL of 1 N HCl and extracted with dichloromethane (4.times.50 mL). The organic phase was washed with brine, dried with Mg₂SO₄, filtered through a plug of activated charcoal and silica gel, and evaporated under reduced pressure. The resulting oil was crystallized from ethanol/water to form a white powder. Yield: 28.98 g (84%). mp 75-76° C. IR: 3449 (br), 1601, 1484, 1275, 1152,834 cm^{-1 1}H NMR (CDCl₃, TMS) d 7.70 (d,J=7.50 Hz, 2H), 7.308-7.235 (m, 3 H), 6.937 (t, J=7.79 Hz, 1 H), 6.682-6.660 (m, 1 H), 6.427-6.404 (m, 2 H), 4.926 (d, J=3.73 Hz, 1 H), 2.936 (d,J=3.76 Hz, 1 H), 2.739-2.610 (m, 4 H), 1.942-1.879 (m, 2 H), 0.935 (s, 9 H), 0.111 (s, 6 H). ¹³C NMR (CDCl₃, TMS) d 154.43, 138.89, 137.47, 130.42, 128.00, 127.69, 127.36, 121.23, 119.89, 119.54, 80.74, 66.36, 27.22, 26.93, 25.65, 24.69, 18.03, -4.40. Anal. Calcd. C₂₃H₃₂O₂S₂ Si: C, 63.84; H, 7.45. Found: C, 63.83; H, 7.26.

### Example 3

### Synthesis of (±)-1-hydroxy-1-(3-carbomethoxymethoxyphenyl)-2-phenyl-2-(1,3-dithian-2 -yl)-ethane (FIG. 1, Compound 3):

The phenolic hydroxyl was conveniently and selectively alkylated by treatment with TBAF in the presence of methyl bromoacetate, to yield the methyl ester. A solution of (±)-1-hydroxy-1-(3-tert-butyldimethylsilyloxyphenyl)-2-phenyl-2-(1,3-di thian-2-yl)-ethane (28.12 g, 65 mmol) and methyl bromoacetate (12.43 g, 81.25 mmol) in 150 mL dry THF was prepared under a nitrogen atmosphere. The solution was treated with 1 M TBAF in THF (68.25 mL, 68.25 mmol) dropwise. The solution was allowed to react overnight, then was poured into ethyl acetate (200 mL) and washed with water (5.times.50 mL). The organic phase was dried with Mg₂SO₄ and evaporated. The residue was dissolved in 200 mL diethyl ether, filtered through a small quantity of neutral alumina and activated charcoal, and dried in vacuo. The product was crystallized from ethyl acetate/hexanes, to afford a white powder. Yield: 23.61 g (93%). mp 122-122.5° C. IR: 3471 (br), 1760, 1595, 1441, 1211, 714 cm^{-1 1}H NMR(CDCl₃, TMS) d 7.679 (dd,J=8.16, 1.55 Hz, 2 H), 7.265-7.325 (m, 3 H), 7.045 (t,J=7.92 Hz, 1 H), 6.804-6.782 (m, 1 H), 6.553 (d,J=7.61 Hz, 1 H), 6.302 (s, 1 H), 4.960 (d, J=3.51 Hz, 1 H), 4.367 (s, 2 H), 3.778 (s, 3 H), 3.023 (d,J=3.52 Hz), 2.757-2.620 (m, 4 H), 1.951-1.891 (m, 2 H). ¹³C NMR (CDCl₃, TMS)d 169.17, 156.61, 138.88, 137.40, 130.46, 128.08, 127.98, 127.49, 121.76, 115.35, 113.68, 80.73, 66.32, 52.11, 27.30, 26.99, 24.74. Anal. Calcd. for C₂₀H₂₂O₄S₂ : C, 61.51; H, 5.68. Found: C, 61.34; H, 5.75.

### Example 4

### Synthesis of (±)-1-hydroxy-1-(3-carboxymethoxyphenyl)-2-phenyl-2-(1,3-dithian-2-yl)- ethane (FIG. 1, Compound 4):

A solution of anhydrous lithium iodide (2.68 g, 20 mmol, Aldrich) in 25 mL dry pyridine was brought to reflux under a nitrogen atmosphere and treated with (±)-1-hydroxy-1-(3-carbomethoxymethoxyphenyl)-2-phenyl-2-(1,3-dithian-2 -yl)-ethane (1.95 g, 5 mmol). The reaction was refluxed for 6 h, then allowed to cool to room temperature under a stream of nitrogen. The solution was poured into 1 N HCl (300 mL), and extracted with ethyl acetate (3.times.50 mL). The combined ethyl acetate layers were extracted with 5% sodium bicarbonate (4.times.50 mL). The aqueous phase was acidified to pH 2, and extracted with ethyl acetate (3.times.50 mL). The organic phase was dried with Mg₂SO₄, filtered through activated charcoal, evaporated, and triturated with hexanes to yield a white solid. Yield: 1.71 g (91%). mp 99-101° C. IR: 3448 (br), 1735, 1595, 1462, 1232, 719 cm⁻¹. ¹H NMR (CDCl₃, TMS) d 7.656 (dd, J=8.00, 1.71 Hz, 2 H), 7.299-7.254 (m, 3 H), 7.036 (t,J=7.98 Hz, 1 H), 6.794-6.773 (m, 1 H), 6.573 (d,J=7.55 Hz, 1 H), 6.251 (s, 1 H), 4.955 (s, 1 H), 4.357 (s, 2 H), 2.728-2.620 (m, 4 H), 1.914-1.868 (m, 2H). ¹³C NM-R (CDCl₃, TMS) d 173.47, 156.32, 139.01, 137.40, 130.50, 128.12, 128.07, 127.56, 122.02, 115.43, 113.66, 80.56, 66.13, 64.73, 27.26, 26.96, 24.67. Anal. Calcd. for C₁₉H₂₀O₄S₂ : C, 60.62; H, 5.35. Found: C, 60.36; H, 5.21.

### Example 5

### Synthesis of (±)1-hydroxy-1-(3-carbamylmethoxyphenyl)-2-phenyl-2-(1,3-dithian-2-yl)- ethane (FIG. 1, Compound 5):

A solution of (±)-1-hydroxy-1-(3-carboxymethoxyphenyl)-2-phenyl-2-(1,3-dithian-2-yl)- ethane (391 mg, 1 mmol) was prepared in 50 mL methanol with gentle warming. The solution was cooled to 0° C., and gaseous ammonia was bubbled through for 30 min. The flask was wrapped in a towel, securely stoppered, and allowed to come to room temperature. After 2 h, the solvent was removed under reduced pressure to yield a white solid. Yield: 348 mg (93%). mp 140-141°C.

IR: 3460, 3346 (br), 1680, 1586, 1442, 1252, 1058, 714 cm⁻¹. ¹H NMR (CDCl₃, TMS) d 7.686 (dd,J=7.82, 1.70 Hz, 2 H), 7.336-7.291 (m, 3 H), 7.088 (t,J=7.93 Hz, 1 H), 6.774 (dd,J=8.09, 2.55 Hz, 1 H), 6.626 (d,J=7.67 Hz, 1 H), 6.463 (s, br, 1 H), 6.335 (s, 1 H), 5.582 (s, br, 1 H), 4.971 (d,J=3.16 Hz, 1 H), 4.245 (s, 2 H), 3.092 (d J=3.24 Hz, 1 H), 2.777-2.635 (m, 4 H), 1.962-1.905 (m, 2 H). ¹³C NMR (CDCl₃, TMS) d 170.699, 156.059, 139.293, 137.529, 130.437, 128.245, 127.696, 122.261, 114.772, 114.270, 80.706, 67.077, 66.462, 27.342, 27.002, 24.729. Anal. Calcd. for C₁₉H₂₁NO₃S₂ : C, 60.77; H, 5.64; N, 3.73. Found: C, 60.93; H, 5.79; N, 3.76.

### Example 6

### Synthesis of (±)-1-acetoxy-1-(3-carbamylmethoxyphenyl)-2-phenyl-2-(1,3-dithian-2-yl) -ethane (FIG. 1, Compound 6):

To a solution of (±)-1-hydroxy-1-(3-carbamylmethoxyphenyl)-2-phenyl-2-(1,3-dithian-2-yl) -ethane (192 mg, 0.5 mmol) in 10 mL THF was added DMAP (2 mg), triethylamine (70 mL, 0.5 mmol), and acetic anhydride (94 mL, 1.0 mmol). The solution was stirred at room temperature for 4 h, and then partitioned between ethyl acetate (50 mL) and 5% sodium bicarbonate (50 mL). The organic phase was washed with water (3.times.50 mL), dried with Mg₂SO₄, and evaporated to yield a colorless oil. Yield 205 mg (98%). IR: 3479, 3331 (br), 1747, 1694, 1589, 1443, 1224, 1033, 910, 718 cm⁻¹. ¹H NMR (CDCl₃, TMS) d 7.740 (dd, J=8.15, 1.55 Hz, 2 H), 7.348-7.281 (m, 3 H), 7.111 (t,J=7.96 Hz, 1 H), 6.801 (dd, J=8.18, 2.53 Hz, 1 H), 6.686 (d, J=7.59 Hz, 1 H), 6.521 (s, 2 H), 6.312 (s, 1 H), 6.136 (s, 1 H), 4.238 (s, 2 H), 2.751-2.597 (m, 4 H), 2.104 (s, 3 H), 1.933-1.857 (s, 1 H). ¹³C NMR (CDCl₃, TMS) d 171.080, 169.318, 156.012, 1 36.974, 130.771, 128.402, 128.045, 127.738, 122.586, 115.072, 114.635, 79.890, 66.982, 63.133, 27.270, 27.109, 24.568, 20.848. Anal. Calcd. for C.sub.21,H.sub.23 NO.sub.4 S.sub.2 : C, 60.41; H, 5.55; N, 3.35. Found: C, 59.92; H, 5.76; N, 3.14.

### Example 7

### Synthesis of (±)-O-acetyl-3'-carbamylmethoxybenzoin (FIG. 1, Compound 7):

To a solution of (±)-1-acetoxy-1-(3-carbamylmethoxyphenyl)-2-phenyl-2-(1,3-dithian-2-yl)-ethane (110 mg, 0.26 mmol) in 5 mL 9:1 (v/v) acetonitrile/water was added mercuric perchlorate (148 mg, 0.33 mmol). The solution was stirred for 15 min, filtered through a 0.45 mm PTFE syringe filter (Gelman) into a 5% sodium bicarbonate solution (10 mL), and extracted with 50 mL dichloromethane. The organic phase was dried and evaporated under reduced pressure to yield a colorless oil. Samples for analysis were evaporated from methanol, dissolved in warm water and lyophilized. Yield: 65 mg (76%). IR: 3445, 1743, 1694, 1462, 1236, 1075, 720 cm⁻¹. ¹H NMR(CDCl₃, TMS)d 7.936 (d,J=7.82 Hz, 2 H), 7.529 (t,J=7.56 Hz, 1 H), 7.412 (t,J=7.57 Hz, 2 H), 7.319 (t,J=7.86 Hz, 1 H), 7.139 (d, J=7.51 Hz, 1 H), 7.051 (s, 1 H), 6.884 (dd, J=8.21, 2.75 Hz, 1 H) 6.835 (s, 1 H), 6.558 (s, 1 H), 6.148 (s, 1 H), 4.462 (s, 2 H), 2.207 (s, 3 H). ¹³C NMR (CDCl₃, TMS) d 193.577, 170.825, 170.348, 157.593, 135.473, 134.494, 133.637, 130.516, 128.768, 125.711, 122.371, 115.205, 115.140, 77.144, 67.115, 20,715. Anal. Calcd. for C₁₈H₁₇NO₅H₂O: C, 62.59; H, 5.54; N, 4.05. Found: C, 62.53; H, 5.12; N, 3.90.

### Steady-state Photolysis of (±)-O-acetyl-3'-carbamylmethoxybenzoin:

A 47.7 mM solution of (±)-O-acetyl-3'-carbamylmethoxybenzoin in 1:1 methanol/Tris.HCl (0.05 M, pH 7.40) was prepared in a 1-cm pathlength quartz cuvette. The sample was irradiated by an Oriel 66011 Hg vapor lamp operating at 450 watts, filtered with a water-cooled Schott glass UG11 filter. At intervals, the sample was removed, and the UV absorption spectrum from 210-400 nm taken by a HP 8452 spectrophotometer. Complete photolysis occurred within a 90 sec exposure.

For isolation of the photoproduct, a 25.6 mg sample of (±)-O-acetyl-3'-carbamylmethoxybenzoin in 50 mL methanol was irradiated in 3 mL batches as above, until no further change was observed in the absorption spectrum of the sample. The methanol was removed under reduced pressure to yield 20.6 mg (99%) of the photoproduct. The composition of this material was 74% 2-phenyl-5-carbamylmethoxybenzofuran, 24% 2-phenyl-7-carbamylmethoxybenzofuran, and 2% other material, as determined by GCMS. Standard samples were obtained by preparative TLC (silica gel/diethyl ether) of the crude photolyzed sample, and identified by ¹H NMR and IR.

### Transient Photolysis of (±)-O-acetyl-3'-carbamylmethoxybenzoin:

A 9.54 mM solution of (±)-O-acetyl-3'-carbamylmethoxybenzoin in 1:1 methanol/Tris.HCl (0.05 M, pH 7.40) was prepared in a 1-cm pathlength quartz cuvette. The sample was photolyzed using the third harmonic at 355 nm from a Q-switched Spectra Physics DCR-12 Nd:YAG laser. Typical pulses were 10-20 ns (FWHM) in duration at an energy of 1.5 mJ/pulse. The sample was monitored with a 75-W xenon arc lamp filtered with a Schott glass UG11 filter placed between the arc lamp and the cuvette. The probe light exiting the cuvette was then wavelength selected by a SA 1690B double monochromator set at 310 nm, and was detected with a photomultiplier. The signal was amplified with a Keithly 427 current amplifier, and digitized by a Tektronix R₇ 10 200 MHz transient digitizer interfaced to a microcomputer. Samples were acquired at a 10 Hz photolysis pulse repetition rate, and scans represented the average of 20 pulses.

Irradiation of (±)-O-acetyl-3'-carbamylmethoxybenzoin resulted in a clean conversion to the phenylbenzofurans (FIG. 1, Compounds 8 and 9). Steady-state photolysis spectra of (±)-O-acetyl-3'-carbamylmethoxybenzoin show two isosbestic points throughout the course of the photolysis (FIG. 3). The two isomeric photoproducts, Compounds 8 and 9 of FIG. 1, were produced in a 98% yield at a ratio of 3:1 as determined by GCMS and NMR of the isolated phenylbenzofurans, along with an equivalent of acetic acid.

Due to the large absorption change at 310 nm (Δε = 35,000 M⁻¹ cm⁻¹), transient absorption studies of the formation of the benzofurans were performed. Quenching studies by Sheehan et al. supra, showed that photolysis of 3',5'-dimethoxybenzoin acetate is extremely rapid, with a rate on the order of 10¹⁰ sec⁻¹. Rapid photolysis seems to have been preserved in the (±)-O-acetyl-3'-carbamylmethoxybenzoin. Photolysis of (±)-O-acetyl-3'-carbamylmethoxybenzoin with a frequency tripled Nd:YAG laser at 355 nm resulted in a rapid absorption increase at 310 nm. The course of this increase could not be measured within the instrument response time of approximately 30 ns, which places a lower limit on the photolysis rate of 3 x 10⁷sec⁻¹ (data not shown).

### Example 8

### Dithiane-protected 3',5'-dimethoxybenzoin:

A solution of 2-phenyl-1,3-dithiane (390 mg, 2 mM) in 20 mL of dry THF was cooled to 0° C. and 1.01 equivalents of nBuLi was added dropwise via syringe with rapid stirring. This solution was allowed to stir for 30 minutes and then 1.0 equivalents of 3,5-dimethoxybenzaldehyde dissolved in 1 ml dry THF was added dropwise. The solution was allowed to warm to room temperature and stir for 1 hr. The reaction was quenched by the addition of aqueous NH₄Cl, THF solvent is removed in vacuo and the resultant slurry extracted with dichloromethane. The dichloromethane layer was washed with 2.times.20 mL of water and solvent removed in vacuo to yield a pale yellow oil. The obtained oils typically crystallize upon standing and are greater than 99% pure based on GC/MS. ¹H NMR and TLC. As such, they can be used for further synthetic transformations without puri fication.

### Example 9

### Dithiane-protected 4'-methoxybenzoin:

The procedure in Example 8 was followed except that 1.0 equivalent of 4-methoxybenzaldehyde was used in place of the 3,5-dimethoxybenzaldehyde.

### Example 10

### Dithiane-protected 2'-ethoxybenzoin:

The procedure in Example 8 was followed except that 1.0 equivalent of 2-ethoxybenzaldehyde was used in place of the 3,5-dimethoxybenzaldehyde.

### Example 11

### Dithiane-protected 2'-methylbenzoin:

The procedure in Example 8 was followed except that 1.0 equivalent of 2-methylbenzaldehyde was used in place of the 3,5-dimethoxybenzaldehyde.

### Example 12

### Synthesis of 3',5'-dimethoxybenzoinyl ethyl carbonate:

A solution of 3.927 g (20 mmol) 2-phenyl-1,3-dithiane in 100 mL dry THF under a nitrogen atmosphere at 0° C. was prepared. N-butyllithium (8 mL, 2.5 M in hexanes, 20 mmol) was added dropwise over 5 minutes, and the solution stirred for one-half hour. Solid 3,5-dimethoxybenzaldehyde (3.324 g, 20 mmol) was then added. After one-half hour, ethyl chloroformate (2.713 g, 25 mmol) was added. Additional THF was added (100 mL), followed by a solution of 18.42 g (41 mmol) mercuric perchlorate dissolved in a minimal quantity of water. After 15 minutes, a solution of 5.560 g K₂CO₃ in minimal water was added and the mixture filtered through a plug of silica gel. Diethyl ether (200 mL) was added to the filtrate and the solution extracted with 5% (w/v) NaHCO₃ and brine. The organic layer was dried with Mg₂SO₄, filtered and evaporated. Crystallization from diethyl ether/hexanes afforded 6.04 g (88%) of the title compound. This material was recrystallized from diethyl ether to yield 4.20 g (61%) of analytically pure material. ¹H H-NMR (CDCl₃, TMS) delta.7.938 (m, 2 H), 7.523 (m, 1 H), 7.404 (m, 2 H), 6.654 (s, 1 H), 6.615 (d,J=2.2 Hz, 2 H), 6.409 (t, J=2.2 Hz, 1H), 4.228 (q,J=6.3 Hz, 2 H) 3.744 (s, 6 H), 1.323 (t, J=2.2 Hz, 3 H).

The foregoing description details specific methods that can be employed to practice the present invention. Having detailed such specific methods, those skilled in the art will well enough know how to devise alternative reliable methods at arriving at the same information in using the fruits of the present invention. Thus, however, detailed the foregoing may appear in text, it should not be construed as limiting the overall scope thereof; rather, the ambit of the present invention is to be determined only by the lawful construction of the appended claims.

## Claims

1. A photoresponsive fragrance composition comprising:
(A) a compound having the formula 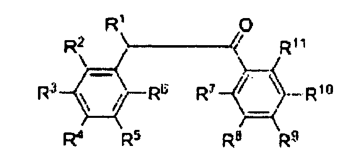 wherein
R₁ is a fragrance molecule; R2 is hydrogen, hydroxy, alkyl, aryl, alkoxy or substituted alkoxy;
R₃, R₄, R₅, and R₆ are independently hydrogen, hydroxy, alkyl, aryl, alkoxy or substituted alkoxy;
R₇, R₈, R₉, R₁₀ and R₁₁ are independently hydrogen, alkyl, aryl, alkoxy or substituted alkoxy;
wherein at least one of R₂ or R₆ is hydrogen;
wherein at least one of R₂, R₃, R₄, R₅, or R₆ is hydroxy or substituted alkoxy; and
(B) a carrier for topical administration or direct administration to article of clothing and other textiles or via routine washing.

2. A photoresponsive fragrance composition according to claim 1, wherein
R₁ is substituted ester (--OC (O) R₁₂) , substituted phosphate (--OP(O)(R₁₉)(OR₂₀), substituted thiol (--S-R₁₂) or substituted amine (--NH-R₁₂);
R₁₂ is a fragrance or molecule that imparts a fragrance; and
R₁₉ is a hydrogen and R₂₀ is a fragrance or molecule that imparts a fragrance or R₁₉ is a fragrance or molecule that imparts a fragrance and R₂₀ is a hydrogen.

3. A photoresponsive fragrance composition according to claim 1 or 2, wherein
R₂ is hydrogen, hydroxy, (C₁₋₂₀)alkyl, (C₅₋₁₂) aryl, (C₁₋₂₀)alkoxy or substituted (C₁₋₂₀)alkoxy;
R₃ is hydrogen, hydroxy, (C₁₋₂₀)alkyl, (C₅₋₁₂)aryl, (C₁₋₂₀)alkoxy or substituted (C₁₋₂₀)alkoxy;
R₄ is hydrogen, hydroxy, (C₁₋₂₀)alkyl, (C₅₋₁₂)aryl, (C₁₋₂₀)alkoxy or substituted (C₁₋₂₀)alkoxy;
R₅ is hydrogen, hydroxy, (C₁₋₂₀)alkyl, (C₅₋₁₂)aryl, (C₁₋₂₀)alkoxy or substituted (C₁₋₂₀)alkoxy;
R₆ is hydrogen, hydroxy, (C₁₋₂₀)alkyl, (C₅₋₁₂)aryl, (C₁₋₂₀)alkoxy or substituted (C₁₋₂₀)alkoxy;
R₇ is hydrogen, (C₁₋₂₀)alkyl, (C₅₋₁₂)aryl, (C₁₋₂₀)alkoxy or substituted (C₁₋₂₀)alkoxy;
R₈ is hydrogen, (C₁₋₂₀)alkyl, (C₅₋₁₂)aryl, (C₁₋₂₀)alkoxy or substituted (C₁₋₂₀)alkoxy;
R₉ is hydrogen, (C₁₋₂₀)alkyl, (C₅₋₁₂)aryl, (C₁₋₂₀)alkoxy or substituted (C₁₋₂₀)alkoxy;
R₁₀ is hydrogen, (C₁₋₂₀)alkyl, (C₅₋₁₂)aryl, (C₁₋₂₀)alkoxy or substituted (C₁₋₂₀)alkoxy; and
R₁₁ is hydrogen, (C₁₋₂₀)alkyl, (C₅₋₁₂)aryl, (C₁₋₂₀)alkoxy or substituted (C₁₋₂₀)alkoxy.

4. A composition useful for releasing a desirable fragrance comprising:
(A) a compound having the formula 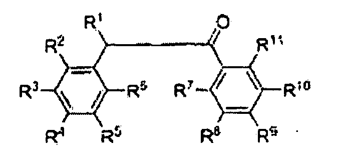 wherein
R₁ is a fragrance molecule; R2 is hydrogen, hydroxy, alkyl, aryl, alkoxy or substituted alkoxy;
R₃, R₄, R₅, and R₆ are independently hydrogen, hydroxy, alkyl, aryl, alkoxy or substituted alkoxy;
R₇, R₈, R₉, R₁₀ and R₁₁ are independently hydrogen, alkyl, aryl, alkoxy or substituted alkoxy;
wherein at least one of R₂ or R₆ is hydrogen;
wherein at least One of R₂, R₃, R₄, R₅, or R₆ is hydroxy or substituted alkoxy; and
(B) a carrier for topical administration or direct administration to article of clothing and other textiles or via routine washing.

5. A composition according to claim 4, wherein
R₁ is substituted ester (--OC(O)R₁₂), substituted phosphate (--OP(O)(R₁₉)(OR₂₀), substituted thiol (--S-R₁₂) or substituted amine (--NH-R₁₂);
R₁₂ is a fragrance or fragrance molecule;
R₁₉ is hydrogen or a fragrance molecule; and
R₂₀ is hydrogen or a fragrance molecule wherein at least one of R₂ or R₆ is hydrogen; wherein at least one of R₂, R₃, R₄, R₅ or R₆ is hydroxy or substituted alkoxy.

6. The composition according to claim 4 or 5 further comprising an auxiliary fragrance agent having a different fragrance than the photoreleased fragrance.

7. The composition according to claim 4 or 5 comprising a photo rearranged product having a desirable fragrance.

8. A composition according to claim 4 or 5, wherein
R₁ is substituted ester (--OC(O)R₁₂), substituted phosphate (--OP(O) (R₁₉)(OR₂₀), substituted thiol (--S-R₁₂), substituted amine (--NH-R₁₂) ;
R₂ is hydrogen, hydroxy, (C₁₋₂₀) alkyl , (C₅₋₁₂) aryl , (C₁₋₂₀)alkoxy or substituted (C₁₋₂₀)alkoxy;
R₃ is hydrogen, hydroxy, (C₁₋₂₀)alkyl, (C₅₋₁₂)aryl, (C₁₋₂₀)alkoxy or substituted (C₁₋₂₀)alkoxy;
R₄ is hydrogen, hydroxy, (C₁₋₂₀)alkyl, (C₅₋₁₂)aryl, (C₁₋₂₀)alkoxy or substituted (C₁₋₂₀)alkoxy;
R₅ is hydrogen, hydroxy, (C₁₋₂₀)alkyl, (C₅₋₁₂)aryl, (C₁₋₂₀)alkoxy or substituted (C₁₋₂₀)alkoxy;
R₆ is hydrogen, hydroxy, (C₁₋₂₀)alkyl, (C₅₋₁₂)aryl, (C₁₋₂₀)alkoxy, or substituted (C₁₋₂₀)alkoxy;
R₇ is hydrogen, (C₁₋₂₀)alkyl, (C₅₋₁₂)aryl, (C₁₋₂₀)alkoxy, or substituted (C₁₋₂₀)alkoxy;
R₈ is hydrogen, (C₁₋₂₀)alkyl, (C₅₋₁₂)aryl, (C₁₋₂₀)alkoxy, or substituted (C₁₋₂₀)alkoxy;
R₉ is hydrogen, (C₁₋₂₀)alkyl, (C₅₋₁₂)aryl, (C₁₋₂₀)alkoxy, or substituted (C₁₋₂₀)alkoxy;
R₁₀ is hydrogen, (C₁₋₂₀)alkyl, (C₅₋₁₂) aryl, (C₁₋₂₀)alkoxy, or substituted (C₁₋₂₀)alkoxy; and
R₁₁ is hydrogen, (C₁₋₂₀)alkyl, (C₅₋₁₂)aryl, (C₁₋₂₀)alkoxy, or substituted (C₁₋₂₀)alkoxy.

9. A composition according to any one of claims 1 to 8, wherein the composition is formulated for spraying application onto the skin.

10. A composition according to any one of claims 1 to 8, wherein the composition is formulated for application onto the hair.

11. A composition according to any one of claims 1 to 9, wherein the compound is of the formula:

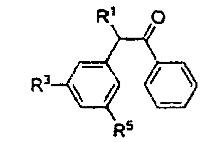

12. A method of controlling the release of a fragrance by incorporating the compositions of any one of claims 1 to 8 or 11 into clothing or textiles by spraying the composition onto the clothing or textiles or washing the clothing or textiles in a solution of the composition.

13. A method according to claim 12, wherein the composition is sprayed onto the clothing or textiles.

14. A method according to claim 12, wherein the composition is incorporated into the clothing or textiles via washing.

## Patentansprüche

1. Eine lichtempfindliche Duftzusammensetzung, die umfasst:
(A) eine Verbindung mit der Formel 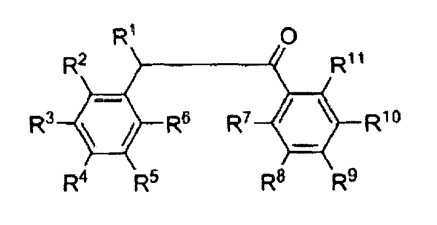 wobei
R₁ ein Duft-Molekül ist; R₂ Wasserstoff, Hydroxy, Alkyl, Aryl, Alkoxy oder substituiertes Alkoxy ist;
R₃, R₄, R₅ und R₆ unabhängig Wasserstoff, Hydroxy, Alkyl, Aryl, Alkoxy oder substituiertes Alkoxy sind;
R₇, R₈, R₉, R₁₀ und R₁₁ unabhängig Wasserstoff, Alkyl, Aryl, Alkoxy oder substituiertes Alkoxy sind;
wobei mindestens eines von R₂ oder R₆ Wasserstoff ist;
wobei mindestens eines von R₂, R₃, R₄, R₅ oder R₆ Hydroxy oder substituiertes Alkoxy ist; und
(B) einen Träger für topische Verabreichung oder direktes Applizieren auf Kleidungsartikel oder andere Textilien oder über regelmäßiges Waschen.

2. Eine lichtempfindliche Duftzusammensetzung gemäß Anspruch 1, wobei
R₁ substituierter Ester (--OC(O)R₁₂), substituiertes Phosphat (--OP(O)(R₁₉)(OR₂₀), substituiertes Thiol (--S-R₁₂) oder substituiertes Amin (--NH-R₁₂) ist;
R₁₂ ein Duft oder Molekül, das einen Duft verleiht, ist; und
R₁₉ ein Wasserstoff ist und R₂₀ ein Duft oder Molekül, das einen Duft verleiht, ist oder R₁₉ ein Duft oder ein Molekül, das einen Duft verleiht, ist und R₂₀ ein Wasserstoff ist.

3. Eine lichtempfindliche Duftzusammensetzung gemäß Anspruch 1 oder 2, wobei
R₂ Wasserstoff, Hydroxy, (C₁₋₂₀) Alkyl, (C₅₋₁₂) Aryl , (C₁₋₂₀) Alkoxy oder substituiertes (C₁₋₂₀) Alkoxy ist;
R₃ Wasserstoff, Hydroxy, (C₁₋₂₀) Alkyl, (C₅₋₁₂) Aryl, (C₁₋₂₀) Alkoxy oder substituiertes (C₁₋₂₀) Alkoxy ist;
R₄ Wasserstoff, Hydroxy, (C₁₋₂₀) Alkyl, (C₅₋₁₂) Aryl, (C₁₋₂₀) Alkoxy oder substituiertes (C₁₋₂₀) Alkoxy ist;
R₅ Wasserstoff, Hydroxy, (C₁₋₂₀) Alkyl, (C₅₋₁₂) Aryl, (C₁₋₂₀) Alkoxy oder substituiertes (C₁₋₂₀) Alkoxy ist;
R₆ Wasserstoff, Hydroxy, (C₁₋₂₀) Alkyl, (C₅₋₁₂) Aryl, (C₁₋₂₀) Alkoxy oder substituiertes (C₁₋₂₀) Alkoxy ist;
R₇ Wasserstoff, (C₁₋₂₀) Alkyl, (C₅₋₁₂) Aryl, (C₁₋₂₀) Alkoxy oder substituiertes (C₁₋₂₀) Alkoxy ist;
R₈ Wasserstoff, (C₁₋₂₀) Alkyl, (C₅₋₁₂) Aryl, (C₁₋₂₀) Alkoxy oder substituiertes (C₁₋₂₀) Alkoxy ist;
R₉ Wasserstoff, (C₁₋₂₀) Alkyl, (C₅₋₁₂) Aryl, (C₁₋₂₀) Alkoxy oder substituiert (C₁₋₂₀) Alkoxy ist;
R₁₀ Wasserstoff, (C₁₋₂₀) Alkyl, (C₅₋₁₂) Aryl, (C₁₋₂₀) Alkoxy oder substituiertes (C₁₋₂₀) Alkoxy ist; und
R₁₁ Wasserstoff, (C₁₋₂₀) Alkyl, (C₅₋₁₂) Aryl, (C₁₋₂₀) Alkoxy oder substituiertes (C₁₋₂₀) Alkoxy ist.

4. Eine Zusammensetzung, die für das Freisetzen eines gewünschten Dufts verwendbar ist, die umfasst:
(A) eine Verbindung mit der Formel 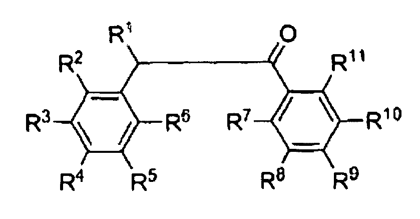 wobei
R₁ ein Duft-Molekül ist; R₂ Wasserstoff, Hydroxy, Alkyl, Aryl, Alkoxy oder substituiertes Alkoxy ist;
R₃, R₄, R₅ und R₆ unabhängig Wasserstoff, Hydroxy, Alkyl, Aryl, Alkoxy oder substituiertes Alkoxy sind;
R₇, R₈, R₉, R₁₀ und R₁₁ unabhängig Wasserstoff, Alkyl, Aryl, Alkoxy oder substituiertes Alkoxy sind;
wobei mindestens eines von R₂ oder R₆ Wasserstoff ist;
wobei mindestens eines von R₂, R₃, R₄, R₅ oder R₆ Hydroxy oder substituiertes Alkoxy ist; und
(B) einen Träger für topische Verabreichung oder direktes Applizieren auf Kleidungsartikel oder andere Textilien oder über regelmäßiges Waschen.

5. Eine Zusammensetzung gemäß Anspruch 4, wobei
R₁ substituierter Ester (--OC(O)R₁₂), substituiertes Phosphat (--OP(O)(R₁₉)(OR₂₀), substituiertes Thiol (--S-R₁₂) oder substituiertes Amin (--NH-R₁₂) ist;
R₁₂ ein Duft oder ein Duft-Molekül ist;
R₁₉ Wasserstoff oder ein Duft-Molekül ist; und
R₂₀ Wasserstoff oder ein Duft-Molekül ist, wobei mindestens eines von R₂ oder R₆ Wasserstoff ist; wobei mindestens eines von R₂, R₃, R₄, R₅ oder R₆ Hydroxy oder substituiertes Alkoxy ist.

6. Die Zusammensetzung gemäß Anspruch 4 oder 5, die weiter ein Hilfsduftmittel umfasst, dass einen unterschiedlichen Duft hat als der durch Licht freigesetzte Duft.

7. Die Zusammensetzung gemäß Anspruch 4 oder 5, die ein durch Licht umgestaltetes Produkt, das einen wünschenswerten Duft hat, umfasst.

8. Eine Zusammensetzung gemäß Anspruch 4 oder 5, wobei
R₁ substituierter Ester (--OC(O)R₁₂), substituiertes Phosphat (--OP(O)(R₁₉)(OR₂₀), substituiertes Thiol (--S-R₁₂), substituiertes Amin (--NH-R₁₂) ist;
R₂ Wasserstoff, Hydroxy, (C₁₋₂₀) Alkyl, (C₅₋₁₂) Aryl, (C₁₋₂₀) Alkoxy oder substituiertes (C₁₋₂₀) Alkoxy ist;
R₃ Wasserstoff, Hydroxy, (C₁₋₂₀) Alkyl, (C₅₋₁₂) Aryl, (C₁₋₂₀) Alkoxy oder substituiertes (C₁₋₂₀) Alkoxy ist;
R₄ Wasserstoff, Hydroxy, (C₁₋₂₀) Alkyl, (C₅₋₁₂) Aryl, (C₁₋₂₀) Alkoxy oder substituiertes (C₁₋₂₀) Alkoxy ist;
R₅ Wasserstoff, Hydroxy, (C₁₋₂₀) Alkyl, (C₅₋₁₂) Aryl, (C₁₋₂₀) Alkoxy oder substituiertes (C₁₋₂₀) Alkoxy ist;
R₆ Wasserstoff, Hydroxy, (C₁₋₂₀) Alkyl, (C₅₋₁₂) Aryl, (C₁₋₂₀) Alkoxy oder substituiertes (C₁₋₂₀) Alkoxy ist;
R₇ Wasserstoff, (C₁₋₂₀) Alkyl, (C₅₋₁₂) Aryl, (C₁₋₂₀) Alkoxy oder substituiertes (C₁₋₂₀) Alkoxy ist;
R₈ Wasserstoff, (C₁₋₂₀) Alkyl, (C₅₋₁₂) Aryl, (C₁₋₂₀) Alkoxy oder substituiertes (C₁₋₂₀) Alkoxy ist;
R₉ Wasserstoff, (C₁₋₂₀) Alkyl, (C₅₋₁₂) Aryl, (C₁₋₂₀) Alkoxy oder substituiert (C₁₋₂₀) Alkoxy ist;
R₁₀ Wasserstoff, (C₁₋₂₀) Alkyl, (C₅₋₁₂) Aryl, (C₁₋₂₀) Alkoxy oder substituiertes (C₁₋₂₀) Alkoxy ist; und
R₁₁ Wasserstoff, (C₁₋₂₀) Alkyl, (C₅₋₁₂) Aryl, (C₁₋₂₀) Alkoxy oder substituiertes (C₁₋₂₀) Alkoxy ist.

9. Eine Zusammensetzung gemäß einem der Ansprüche 1 bis 8, wobei die Zusammensetzung für Sprühende Anwendung auf die Haut formuliert ist.

10. Eine Zusammensetzung gemäß einem der Ansprüche 1 bis 8, wobei die Zusammensetzung für Anwendung auf dem Haar formuliert ist.

11. Eine Zusammensetzung gemäß einem der Ansprüche 1 bis 9, wobei die Verbindung von der Formel 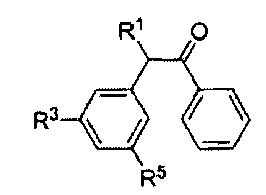 ist.

12. Ein Verfahren zur Steuerung der Freisetzung eines Dufts durch Einfügen der Zusammensetzungen nach einem der Ansprüche 1 bis 8 oder 11 in Kleidung oder Textilien durch Sprühen der Zusammensetzung auf die Kleidung oder Textilien oder Waschen der Kleidung oder Textilien in einer Lösung der Zusammensetzung.

13. Ein Verfahren gemäß Anspruch 12, wobei die Zusammensetzung auf die Kleidung oder Textilien gesprüht wird.

14. Ein Verfahren gemäß Anspruch 12, wobei die Zusammensetzung in die Kleidung oder Textilien durch Waschen eingefügt wird.

## Revendications

1. Composition de parfum photosensible comprenant : (A) un composé ayant la formule : 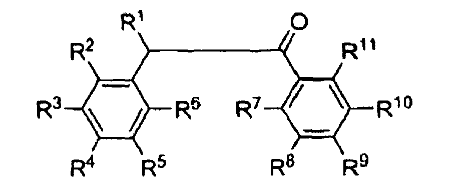 dans laquelle :
R₁ est une molécule de parfum ; R₂ est un groupe hydrogène, hydroxy, alkyle, aryle, alcoxy ou alcoxy substitué ;
R₃, R₄, R₅ et R₆ sont indépendamment un groupe hydrogène, hydroxy, alkyle, aryle, alcoxy ou alcoxy substitué ;
R₇, R₈, R₉, R₁₀ et R₁₁ sont indépendamment un groupe hydrogène, alkyle, aryle, alcoxy ou alcoxy substitué ;
dans laquelle au moins l'un parmi R₂ ou R₆ est un atome d'hydrogène ;
dans laquelle au moins l'un parmi R₂, R₃, R₄ , R₅ ou R₆ est un groupe hydroxy ou un groupe alcoxy substitué ; et
(B) un support destiné à une administration topique ou directe sur un article de vêtement et d'autres textiles ou par lavage de routine.

2. Composition de parfum photosensible selon la revendication 1, dans laquelle :
R₁ est un ester substitué (--OC (O) R₁₂), un phosphate substitué (-OP(O) (R₁₉) (OR₂₀), un thiol substitué (--S- R₁₂) ou une amine substituée (-NH-R₁₂) ;
R₁₂ est un parfum ou une molécule qui confère un parfum ; et
R₁₉ est un atome d'hydrogène et R₂₀ est un parfum ou une molécule qui confère un parfum ou R₁₉ est un parfum ou une molécule qui confère un parfum et R₂₀ est un atome d'hydrogène.

3. Composition de parfum photosensible selon la revendication 1 ou 2, dans laquelle :
R₂ est un groupe hydrogène, hydroxy, alkyle (C₁₋₂₀), aryle (C₅₋₁₂), alcoxy (C₁₋₂₀) ou alcoxy substitué (C₁₋₂₀) ;
R₃ est un groupe hydrogène, hydroxy, alkyle (C₁₋₂₀), aryle (C₅₋₁₂), alcoxy (C₁₋₂₀) ou alcoxy substitué (C₁₋₂₀) ;
R₄ est un groupe hydrogène, hydroxy, alkyle (C₁₋₂₀), aryle (C₅₋₁₂), alcoxy (C₁₋₂₀) ou alcoxy substitué (C₁₋₂₀) ;
R₅ est un groupe hydrogène, hydroxy, alkyle (C₁₋₂₀), aryle (C₅₋₁₂), alcoxy (C₁₋₂₀) ou alcoxy substitué (C1-20) ;
R₆ est un groupe hydrogène, hydroxy, alkyle (C₁₋₂₀), aryle (C₅₋₁₂), alcoxy (C₁₋₂₀) ou alcoxy substitué (C₁₋₂₀) ;
R₇ est un groupe hydrogène, alkyle (C₁₋₂₀), aryle (C₅₋₁₂), alcoxy (C₁₋₂₀) ou alcoxy substitué (C₁₋₂₀) ;
R₈ est un groupe hydrogène, alkyle (C₁₋₂₀), aryle (C₅₋₁₂), alcoxy (C₁₋₂₀) ou alcoxy substitué (C₁₋₂₀) ;
R₉ est un groupe hydrogène, alkyle (C₁₋₂₀), aryle (C₅₋₁₂), alcoxy (C₁₋₂₀) ou alcoxy substitué (C₁₋₂₀) ;
R₁₀ est un groupe hydrogène, alkyle (C₁₋₂₀), aryle (C₅₋₁₂), alcoxy (C₁₋₂₀) ou alcoxy substitué (C₁₋₂₀) et
R₁₁ est un groupe hydrogène, alkyle (C₁₋₂₀), aryle (C₅₋₁₂), alcoxy (C₁₋₂₀) ou alcoxy substitué(C₁₋₂₀) ;

4. Composition utile pour libérer un parfum souhaitable comprenant :
(A) un composé ayant la formule : 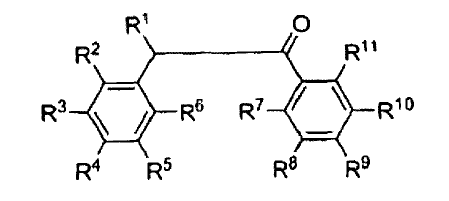 dans laquelle :
R₁ est une molécule de parfum ; R₂ est un groupe hydrogène, hydroxy, alkyle, aryle, alcoxy ou alcoxy substitué ;
R₃, R₄, R₅ et R₆ sont indépendamment un groupe hydrogène, hydroxy, alkyle, aryle, alcoxy ou alcoxy substitué ;
R₇, R₈, R₉, R₁₀ et R₁₁ sont indépendamment un groupe hydrogène, alkyle, aryle, alcoxy ou alcoxy substitué ;
dans laquelle au moins l'un parmi R₂ ou R₆ est un atome d'hydrogène ;
dans laquelle au moins l'un parmi R₂, R₃, R₄, R₅ ou R₆ est un groupe hydroxy ou un groupe alcoxy substitué ; et
(B) un support destiné à une administration topique ou directe sur un article de vêtement et d'autres textiles ou par lavage de routine.

5. Composition selon la revendication 4, dans laquelle R₁ est un ester substitué (--OC(O)R₁₂), un phosphate substitué (--OP(O) (R₁₉) (OR₂₀), un thiol substitué (--S-R₁₂) ou une amine substituée (--NH-R₁₂);
R₁₂ est un parfum ou une molécule de parfum ;
R₁₉ est un atome d'hydrogène ou une molécule de parfum ; et
R₂₀ est un atome d'hydrogène ou une molécule de parfum, dans laquelle au moins l'un parmi R₂ ou R₆ est un atome d'hydrogène ; dans laquelle au moins l'un parmi R₂, R₃, R₄, R₅ ou R₆ est un groupe hydroxy ou un groupe alcoxy substitué.

6. Composition selon les revendications 4 ou 5, comprenant en outre un agent de parfum auxiliaire ayant un parfum différent du parfum photolibéré.

7. Composition selon les revendications 4 ou 5, comprenant un produit photo réaménagé ayant un parfum souhaitable.

8. Composition selon la revendication 4 ou 5, dans laquelle :
R₁ est un ester substitué (--OC (O) R₁₂), un phosphate substitué (--OP(O) (R₁₉) (OR₂₀), un thiol substitué (--S-R₁₂), une amine substituée (--NH-R₁₂) ;
R₂ est un groupe hydrogène, hydroxy, alkyle (C₁₋₂₀), aryle (C₅₋₁₂), alcoxy(C₁₋₂₀) ou alcoxy substitué (C₁₋₂₀) ;
R₃ est un groupe hydrogène, hydroxy, alkyle (C₁₋₂₀), aryle (C₅₋₁₂), alcoxy (C₁₋₂₀) ou alcoxy substitué (C₁₋₂₀) ;
R₄ est un groupe hydrogène, hydroxy, alkyle (C₁₋₂₀), aryle (C₅₋₁₂), alcoxy (C₁₋₂₀) ou alcoxy substitué (C₁₋₂₀);
R₅ est un groupe hydrogène, hydroxy, alkyle (C₁₋₂₀), aryle (C₅₋₁₂), alcoxy (C₁₋₂₀) ou alcoxy substitué (C₁₋₂₀) ;
R₆ est un groupe hydrogène, hydroxy, alkyle (C₁₋₂₀), aryle (C₅₋₁₂), alcoxy (C₁₋₂₀) ou alcoxy substitué (C₁₋₂₀) ;
R₇ est un groupe hydrogène, alkyle (C₁₋₂₀), aryle (C₅₋₁₂), alcoxy (C₁₋₂₀) ou alcoxy substitué (C₁₋₂₀) ;
R₈ est un groupe hydrogène, alkyle (C₁₋₂₀), aryle (C₅₋₁₂), alcoxy (C₁₋₂₀) ou alcoxy substitué (C₁₋₂₀) ;
R₉ est un groupe hydrogène, alkyle (C₁₋₂₀), aryle (C₅₋₁₂), alcoxy (C₁₋₂₀) ou alcoxy substitué (C₁₋₂₀) ;
R₁₀ est un groupe hydrogène, alkyle (C₁₋₂₀), aryle (C₅₋₁₂), alcoxy (C₁₋₂₀) ou alcoxy substitué (C₁₋₂₀) ; et
R₁₁ est un groupe hydrogène, alkyle (C₁₋₂₀), aryle (C₅₋₁₂), alcoxy (C₁₋₂₀) ou alcoxy substitué (C₁₋₂₀) ;

9. Composition selon l'une quelconque des revendications 1 à 8, dans laquelle la composition est formulée pour une application par pulvérisation sur la peau.

10. Composition selon l'une quelconque des revendications 1 à 8, dans laquelle le composition est formulée pour une application sur les cheveux.

11. Composition selon l'une quelconque des revendications 1 à 9, dans laquelle le composé a la formule :

12. Procédé de régulation de libération d'un parfum en incorporant les compositions selon l'une quelconque des revendications 1 à 8 ou 11 aux vêtements ou aux textiles en pulvérisant la composition sur les vêtements ou les textiles ou en lavant les vêtements ou les textiles dans une solution de la composition.

13. Procédé selon la revendication 12, dans lequel la composition est pulvérisée sur les vêtements ou les textiles.

14. Procédé selon la revendication 12, dans lequel la composition est incorporée aux vêtements ou aux textiles par lavage.
